# EUROPEAN PATENT APPLICATION

(11) **EP 1 908 828 A1**
(43) Date of publication of application: **09.04.2008**
(21) Application number: 05766151.4
(22) Date of filing: 22.07.2005
(51) Int. Cl.: C12N 15/09, C07K 4/00, C07K 1/10, C07K 17/14

(54) **NOVEL POLYPEPTIDE AND METHOD FOR PRODUCING THE SAME**

(71) Applicant: PHG Corporation, Kyoto 604-0096 (JP)
(72) Inventor: TANIHARA, Masao, L. Parksquare Kyotokamogawa 218, Kyoto-shi, Kyoto 606-8305 (JP)
(74) Representative: von Kreisler Selting Werner
(86) International application number: PCT/JP2005/013511
(87) International publication number: WO 2007/010623

(57) **Abstract**

The novel polypeptide of the present invention contains a peptide unit having an amino acid sequence represented by the following formula (1) and a peptide unit having an amino acid sequence represented by the following formula (2).
-Pro-X-Gly- (1)
-Y-Z-Gly- (2)

In the formula, "X" and "Z" are the same or different, each represents Pro or Hyp, and "Y" represents an amino acid residue having a carboxyl group (e.g., Asp, Glu, and Gla).

The proportion (molar ratio) of the peptide unit (1) relative to the peptide unit (2) [(1)/(2)] is about 99/1 to 1/99. The polypeptide may have an apatite supported thereon. The polypeptide is free from a risk of an infection by a pathogenic organism or an undesirable side effect and is useful as a highly safe biomaterial or biocompatible material. For example, the polypeptide is useful for supplementing, repairing and/or regenerating a biological hard tissue and is also useful for a material of a cosmetic preparation, a food additive, and others.

SEQUENCE LISTING

<110>  Tanihara, Masao

<120>  Novel polypeptide and process for producing the same

<130>  FP-05014PC

<160>  2

<170>  PatentIn version 3.1

<210>  1
<211>  30
<212>  PRT
<213>  Artificial Sequence

<220>
<223>  Inventor: Tanihara, Masao

<220>
<223>  Description of Artificial Sequence:peptide

<220>
<221>  misc_feature
<222>  (1)..(30)
<223>  Xaa is defined as 4Hyp

<400>  1

Pro Xaa Gly Pro Xaa Gly Pro Xaa Gly Pro Xaa Gly Glu Xaa Gly Pro
1               5                   10                  15

Xaa Gly Pro Xaa Gly Pro Xaa Gly Pro Xaa Gly Pro Xaa Gly
            20                  25                  30

<210>  2
<211>  30
<212>  PRT
<213>  Artificial Sequence

<220>
<223>  Description of Artificial Sequence:peptide

<220>
<221>  misc_feature
<222>  (1)..(30)
<223>  Xaa is defined as 4Hyp

<400>  2

Pro Xaa Gly Pro Xaa Gly Pro Xaa Gly Pro Xaa Gly Pro Xaa Gly Pro
1               5                   10                  15

Xaa Gly Pro Xaa Gly Pro Xaa Gly Pro Xaa Gly Pro Xaa Gly
            20                  25                  30

## Description

### TECHNICAL FIELD

The present invention relates to a novel polypeptide that is free from a risk of an infection by a pathogenic organism (or a causative factor) or an undesirable side effect and is useful for accelerating nucleation of an apatite, and a process for producing the same. More specifically, the present invention relates to a novel polypeptide (including a polypeptide having an apatite supported thereon) useful as a highly safe biomaterial or biocompatible material, in particular, useful for supplementing, repairing and/or regenerating a biological hard tissue as well as for adding or mixing to a cosmetic preparation, a food composition, and others, and a process for producing the same.

### BACKGROUND ART

A collagen is a fibrous protein found in all multicellular organisms. The collagen is a main component of skins or bones and occupies 25% of total proteins in mammals. A typical collagen molecular has a rope-like superhelical structure, which is referred to as a triple helical structure, comprising three collagen polypeptide chains. The collagen is particularly rich in proline (Pro) and glycine (Gly). These two amino acid residues are important to form a stable triple helical structure of the collagen.

As methods for using a collagen as a biomaterial, there may be mentioned, for example, a method of grafting or transplanting an intact or lyophilized skin tissue derived from a pig on a skin area damaged by a burn or scald, a method of removing cellular components from a tissue with enzyme treatment, and a method of using a collagen which is solubilized by a treatment with an acidic solution or an enzyme to reconstitute a desirable form. A common preparation method and a common qualitative method are described in Methods Enzymol. (Non-Patent Document 1), Vol. 82, pp. 33 to 64, 1982.

There are various suggestions to utilize a collagen. For example, Japanese Patent Application Laid-Open No. 08-027192 (JP-08-027192A, Patent Document 1) discloses a production process of a collagen derivative for imparting moisture and smoothness to skin, which comprises esterifying and modifying an animal tissue containing a collagen with alcohol, and extracting the modified collagen, as well as a cosmetic base material using the collagen derivative.

Japanese Patent Application Laid-Open No. 07-097454 (JP-07-097454A, Patent Document 2) discloses a production process of a water-soluble crosslinked collagen which shows a high regeneration rate of a triple helical structure after thermal denaturation, and the process comprises subjecting a water-soluble collagen to a crosslinking treatment with a bifunctional alkylene diimidate cross-linker having imide ester groups at both ends of the methylene chain.

Japanese Patent Application Laid-Open No. 08-053548 (JP-08-053548A, Patent Document 3) mentions a matrix of a collagen and a synthetic polymer (a collagen-synthetic polymer matrix) which has a low immunogenicity and is useful for preparation of biocompatible implants utilized for various medical applications, and a production process of the matrix comprises reacting a collagen with a first synthetic hydrophilic polymer to form a collagen-synthetic polymer matrix, and further reacting the collagen-synthetic polymer matrix with a reactant such as a second synthetic hydrophilic polymer, a biologically active substance, a glycosaminoglycan and a derivative thereof, a chemical crosslinking agent, an esterifying agent, an amidating agent, an acylating agent, an amino acid, a polypeptide, or others.

Japanese Patent Application Laid-Open No. 07-278312 (JP-07-278312A, Patent Document 4) discloses a unit material containing a hydrophilic synthetic polymer covalently bonded to a chemically modified collagen which is substantially a nonfiberous form at pH 7. The document discloses that the unit material is particularly useful for ophthalmological devices and optically transparent and has a biocompatibility.

Japanese Patent Application Laid-Open No. 05-000158 (JP-05-000158A, Patent Document 5) discloses a production process of a collagenic membrane-like substance, which comprises crushing a collagen matrix, centrifuging the crushed matrix under a high centrifugal field, homogenizing the resultant precipitate to obtain a paste, casting the paste, and drying the cast paste at a temperature of not higher than 37°C. The document also discloses that the collagen membrane-like substance has a biocompatibility and a non-inflammatory property and is useful as an artificial implantation matter for repairing a tissue.

Japanese Patent Application Laid-Open No. 05-125100 (JP-05-125100A, Patent Document 6) discloses a soluble fish scale collagen having high-purity and a production process thereof, and the process comprises pepsinating an intact or deashed fish scale.

Japanese Patent Application Laid-Open No. 06-228506 (JP-06-228506A, Patent Document 7) discloses a production process of a dry particulate or powdery soluble collagen, which comprises injecting a collagen solution through a nozzle into 70 to 90% ethanol medium to form a strand-like or membranous product, drying the product, and chopping or grinding the dried product.

Japanese Patent Application Laid-Open No. 08-276003 (JP-08-276003A, Patent Document 8) discloses a use of an unbaked single-crystal hydroxyapatite as a material for repairing a biological hard tissue (such as a bone), through attaching the single crystal to at least part of a low antigenic collagen fiber.

Japanese Patent Application Laid-Open No. 08-041425 (JP-08-041425A, Patent Document 9) mentions a method which comprises removing fragments of cells or tissues in a collagen solution and subjecting the residue to an alkali treatment, for removing prion in a collagen derived from an animal or human being, and discloses a collagen obtained by this method.

Moreover, regarding methods for chemical synthesis of collagen analogues, it has been reported that a soluble polyamide having a molecular weight of 16,000 to 21,000 isobtainedbydissolvingp-nitrophenylesterofPro-Ser-Gly or p-nitrophenyl ester of Pro-Ala-Glyin dimethyl formamide, and adding triethylamine thereto, and allowing to stand the mixture for 24 hours (J. Mol. Biol., Vol. 63 (Non-Patent Document 2), pp. 85 to 99, 1972). In this document, the soluble polyamide is estimated to form a triple helical structure based on the circular dichroism spectra. However, there are not referred to properties of the obtainedpolymer.

It also has been reported that a method for obtaining a polyamide, which comprises dissolving a 50-mer peptide containing the sequence Val-Pro-Gly-Val-Gly derived from elastin in dimethylsulfoxide, adding 2 equivalents of 1-ethyl-3-(3-dimethylaminopropyl)-carbodiimide, 1 equivalent of 1-hydroxybenzotriazole, and 1.6 equivalents of N-methylmorpholine thereto, allowing to stand the mixture for 14 days, and dialyzing the resultant mixture with a dialysis membrane (molecular weight cut-off: 50,000) (Int. J. Peptide Protein Res., Vol. 46 (Non-Patent Document 3), pp. 453 to 463, 1995).

Moreover, Japanese Patent Application Laid-Open No. 2003-321500 (JP-2003-321500A, Patent Document 10) discloses that a polypeptide comprising peptide units represented by the following formulae (1) to (3) is capable of forming a collagenous tissue:

[-(OC-(CH₂)ₘ-CO)ₚ-(Pro-Y-Gly)ₙ-]ₐ (1)

[-(OC-(CH₂)ₘ-CO)_{q}-(2)ᵣ-]_{b} (2)

[-HN-R-NH-]_{c} (3)

wherein "m" denotes an integer of 1 to 18, "p" and "q" are the same or different, each representing 0 or 1, "Y" represents Pro or Hyp, and "n" denotes an integer of 1 to 20; "Z" represents a peptide chain comprising 1 to 10 amino acid residue(s), "r" denotes an integer of 1 to 20, and "R" represents a straight chain or branched chain alkylene group; the molar ratio of "a" relative to "b" [a/b] is 100/0 to 30/70;
when p=1 and q=0, c=a,
when p=0 and q=1, c=b,
when p=1 and q=1, c=a+b, and
when p=0 and q=0, c=0.
As described in the above-mentioned Patent Document 9, a causative substance of sheep tremor or bovine spongiform encephalopathy is an infectious protein called as prion, and the infectious protein is considered as one of causes of human Creutzfeldt-Jakob disease infection. Prion is a protein, and it is indicated that prion is hard to deactivate with a conventional pasteurization or sterilization method, further that prion is infectious over species (Nature Review, Vol. 2 (Non-Patent Document 4), pp. 118 to 126, 2001).

In general, a collagen derived from bovine or pig is frequently used as a raw material for medical kits (devices) or pharmaceutical preparations, and cosmetic preparations. Accordingly, there have been always existed the risk of an infection (or a transmission) to pathogenic organisms (or a causative factor) such as prion which cannot be removed by a conventional pasteurization or sterilization.

Moreover, since various cell adhesion sites are found in a naturally occurring collagen, the naturally occurring collagen cannot exert cell selectivity depending on the usage. For example, in the use of a collagen as a material for inducing a nerval axon, migration or growth rate of surrounding fibroblast is higher than elongation rate of the axon resulting in forming scarring tissue, and the axon cannot be elongated. It is therefore necessary to take a step to cover around the collagen with a material for preventing migration of fibroblast, or others.

On the other hand, it has been known that, in a living body, a certain variety of ceramics [for example, Bioglass (registered trademark) as a bioactive glass, and crystallized glass A-W (Cerabone (registered trademark) A-W)] bond to a bone. The bond of the ceramics to the bone is caused by formation of a hydroxyapatite layer on the surface of the ceramics in a living body (or in an aqueous solution having an ion concentration close to that of human body fluids). It is considered that the bonding mechanism is as follows: firstly, a silicate ion or silanol group formed on the surface of the ceramics is reacted with a calcium ion and a phosphate ion in a living body or an aqueous solution to form a nucleus of a hydroxyapatite, and supersaturated calcium and phosphate ions in the living body or the aqueous solution are further incorporated to the nucleus as a basis to glow the hydroxyapatite layer.

Japanese Patent Application Laid-Open No.5-103829 (JP-5-103829A, Patent Document 11) discloses a coating method of a bioactive layer, which comprises coating a liquid silica hydrosol or hydrogel on a substrate (e.g., a metal and a ceramic, each having a variety of forms such as a plate, a rod, a fiber and a particle), drying and heating the coated matter for bonding the silica gel to the substrate, and then immersing the resulting matter in an aqueous solution (a simulated body fluid) containing calcium and phosphate ions at an amount supersaturated to a hydroxyapatite for coating the surface of the substrate with a hydroxyapatite layer. This document also mentions that an apatite-coated material is available for an artificial bone, a biological implantable material, a biological implantable medical equipment, and others. However, in such an inorganic biomaterial, biocompatibility such as cell adhesion is insufficient.

Moreover, an organic-inorganic complex (or composite) material as a biomaterial has been studied. For example, Japanese Patent Application Laid-Open No. 2003-190271 (JP-2003-190271A, Patent Document 12) discloses an organic-inorganic complex (or composite) biomaterial which comprises a complex containing a hydroxyapatite having a mean fiber length of not shorter than 60 µm and a collagen (e.g., a collagen or a collagenous protein obtained from mammals, birds, fishes and others, and a genetically-modified collagen). Further, this document also describes that the complex biomaterial can be produced by maintaining concentrations of a calcium ion and a phosphate ion in a reaction vessel to certain values through, for example, controlling of a concentration of a starting material or a solution-feeding speed, and pressing the obtained complex for molding. Furthermore, the document "Chem. Mater. 15", pp. 3221-3226, 2003" (Non-Patent Document 5) discloses a process for forming a complex of a collagen and a hydroxyapatite, which comprises neutralizing a collagen in the presence of 0.1 M CaCl₂ and 0.1 M NaH₂PO₄, in which the collagen is derived from rat tail tendon solubilized by an acid.

However, even in such a complex, the use of a naturally occurring collagen as the collagen has a risk of an infection (or transmission) of a pathogenic organism (or a causative factor).

Incidentally, Japanese Patent Application Laid-Open No. 154001/2003 (JP-2003-154001A, Patent Document 13) discloses a process for producing a complex, which comprises bringing an aqueous solution containing a calcium ion and a phosphate ion into contact with a base having a sericin to form a complex in which an apatite is precipitated on the base.
[Patent Document 1] JP-08-027192A
[Patent Document 2] JP-07-097454A
[Patent Document 3] JP-08-053548A
[Patent Document 4] JP-07-278312A
[Patent Document 5] JP-05-000158A
[Patent Document 6] JP-05-125100A
[Patent Document 7] JP-06-228506A
[Patent Document 8] JP-08-276003A
[Patent Document 9] JP-08-041425A
[Patent Document 10] JP-2003-321500A (Claim 1 and paragraph number [0043])
[Patent Document 11] JP-5-103829A (Claim 1)
[Patent Document 12] JP-2003-190271A (Claims 1, 4 and 6, and paragraph number [0014])
[Patent Document 13] JP-2003-154001A (Claim 1)
[Non-Patent Document 1] "Methods Enzymol., Vol. 82", pp. 33-64, 1982
[Non-Patent Document 2] "J. Mol. Biol., Vol.63", pp. 85-99, 1972
[Non-Patent Document 3] "Int. J. Peptide Protein Res., Vol.46", pp. 453-463, 1995
[Non-Patent Document 4] "Nature Review, Vol.2", pp. 118-126, 2001
[Non-PatentDocument5] "Chem.Mater.15",pp.3221-3226, 2003

### DISCLOSURE OF THE INVENTION

### PROBLEMS TO BE SOLVED BY THE INVENTION

It is therefore an object of the present invention to provide a novel polypeptide that is free from a risk of an infection by a pathogenic organism and/or a transmission of a causative factor or a risk of an undesirable side effect and is useful as a highly safe biomaterial or biocompatible material, and a process for producing the same.

It is another object of the present invention to provide a novel polypeptide useful as a component of a cosmetic preparation or a food composition, a process for producing the same, and a composition containing the polypeptide.

It is further object of the present invention to provide a polypeptide useful for supplementing, repairing and/or regenerating a biological hard tissue.

### MEANS TO SOLVE THE PROBLEMS

The inventor of the present invention made intensive studies to achieve the above objects and finally found that a collagen-like polypeptide (fiber assembly) obtained by condensing a peptide component containing a specific amino acid sequence deposits a hydroxyapatite thereon in a living body (or in vivo) or under an environment analogous to a living body. The present invention was accomplished based on the above findings.

That is, the novel polypeptide of the present invention contains (1) a peptide unit having an amino acid sequence represented by the following formula (1) and (2) a peptide unit having an amino acid sequence represented by the following formula (2):

-Pro-X-Gly- (1)

-Y-Z-Gly- (2)

wherein "X" and "Z" are the same or different, each representing Pro or Hyp, and "Y" represents an amino acid residue having a carboxyl group (e.g., an α-amino acid residue).
In the formula (2), "Y" may be Asp, Glu, or Glu having a further carboxyl group at γ-position.

The proportion (molar ratio) of the peptide unit (1) relative to the peptide unit (2) [(1)/(2)] may be about 99/1 to 1/99.

The polypeptide of the present invention shows positive Cotton effect at a wavelength in the range of 220 to 230 nm and negative Cotton effect at a wavelength in the range of 195 to 205 nm in a circular dichroism spectrum. This shows that at least part (part or all) of the polypeptide forms a triple helical structure. The polypeptide may show a peak corresponding to the molecular weight in a range from 5 x 10³ to 500 x 10⁴. Moreover, the polypeptide is capable of forming a collagenous tissue (a collagen-like fiber assembly). The polypeptide mayhave an apatite (e.g., a hydroxyapatite) supported thereon.

In the present invention, the polypeptide may be obtained by condensing an amino acid component or peptide fragment component which at least contains an amino acid or peptide fragment corresponding to the formula (1) and an amino acid or peptide fragment corresponding to the formula (2). The polypeptide may be produced by, for example, (a) condensing a peptide component at least containing a peptide having both amino acid sequences represented by the formulae (1) and (2) or (b) condensing a peptide component at least containing a peptide having an amino acid sequence represented by the formula (1) and a peptide having an amino acid sequence represented by the formula (2).

The polypeptide (particularly, a fiber assembly thereof), for example, can deposit or precipitate (or sediment) an apatite (e.g., a hydroxyapatite) under an environment analogous to a body fluid and can form a complex (a hybrid) of the polypeptide and the apatite. In the present invention, a polypeptide having an apatite supported thereon may be produced by bringing the polypeptide into contact with an aqueous solution containing a calcium ion and a phosphate ion to deposit an apatite on the polypeptide. The present invention also includes a polypeptide having an apatite supported thereon obtainable by such a production process.

Moreover, the present invention includes a composition containing the polypeptide (e.g., a cosmetic preparation and a food composition).

### EFFECTS OF THE INVENTION

The novel polypeptide of the present invention is excellent in cytophilic property or biocompatibility due to a specific amino acid sequence thereof, while the polypeptide is free from a risk of an infection of pathogenic organism or a transmission of a causative factor or free from an undesirable side effect and is highly safe. Moreover, the polypeptide (particularly, a fiber assembly of the polypeptide) deposits or precipitates an apatite in a living body or under an environment analogous to a living body (e.g., under an environment analogous to a body fluid) and is capable of forming a complex of the polypeptide and the apatite, and the complex is useful for supplementing, repairing, and/or regenerating a biological hard tissue. Such a polypeptide or such a complex is suitable for a biomaterial or a biocompatible material. Further, the polypeptide or the complex is also useful for a component of a cosmetic preparation or a food composition.

### BRIEF DESCRIPTION OF DRAWINGS

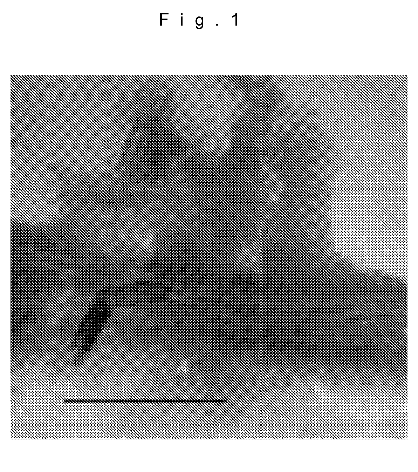

[Fig. 1] Fig. 1 is an electron micrograph of a complex (or a composite) of a polypeptide obtained in Example 1 and an apatite (the scale bar in the figure indicates 100 nm).

### DETAILED DESCRIPTION OF THE INVENTION

In the present invention, amino acid residues are abbreviated to the following condensation codes.

Ala: L-alanine residue
Arg: L-arginine residue
Asn: L-asparagine residue
Asp: L-aspartic acid residue
Cys: L-cysteine residue
Gln: L-glutamine residue
Glu: L-glutamic acid residue
Gly: glycin residue
His: L-histidine residue
Hyp: L-hydroxyproline residue
Ile: L-isoleucine residue
Leu: L-leucine residue
Lys: L-lysine residue
Met: L-methionine residue
Phe: L-phenylalanine residue
Pro: L-proline residue
Sar: sarcosine residue
Ser: L-serine residue
Thr: L-threonine residue
Trp: L-tryptophan residue
Tyr: L-tyrosine residue
Val: L-valine residue
Moreover, in this specification, amino acid sequences of peptide chains are represented in accordance with conventional expression that, in an amino acid residue, N-terminus and C-terminus are drawn at the left and the right, respectively.

### [Novel polypeptide]

It is necessary that the novel polypeptide of the present invention contain a peptide unit having an amino acid sequence represented by the formula -Pro-X-Gly- (1). The sequence represented by the formula -Pro-X-Gly- contributes to the stability of a triple helical structure. Therefore, a low proportion of the sequence decreases the stability of the triple helical structure. Further, from the viewpoint of the stability of the triple helical structure, the unit may form a repeating structure represented by -(Pro-X-Gly)ₙ- (an oligo- or polypeptide unit structure) in the polypeptide. The repeating number "n" of the sequences is, for example, about 1 to 5000 and preferably about 2 to 3000. The group "X" may be either Pro or Hyp. From the viewpoint of the stability of the triple helical structure, the more preferred "X" is Hyp. Incidentally, Hyp is usually 4Hyp (e.g., trans-4-hydroxy-L-proline) residue.

Moreover, it is necessary that the polypeptide of the present invention contain a peptide unit having an amino acid sequence represented by the formula -Y-Z-Gly- (2). Such a peptide unit (2) allows the polypeptide to form or support an apatite (e.g., a hydroxyapatite) efficiently.

In the formula (2), the group "Y" may be an amino acid residue having a carboxyl group (e.g., an α-amino acid residue having a carboxyl group) . Such an amino acid residue may include, for example, Glu and Asp. The Glu may have a carboxyl group at they γ-position. The Glu having a carboxyl group at the γ-position is represented by γ-carboxyglutamic acid residue, Gla. Moreover, in the above-mentioned formula (2), the group "Z" may be either Pro or Hyp.

Further, the polypeptide of the present invention may contain other amino acid residues or peptide residues (units) as far as the resulting polypeptide is not deteriorated in physical and biological properties. In order that the polypeptide of the present invention may show usefulphysicaland biologicalproperties,for example, the polypeptide usually comprises at least one amino acid residue or peptide residue selected from the group consisting of Gly, Sar, Ser, Glu, Asp, Lys, His, Ala, Val, Leu, Arg, Pro, Tyr, Ile, Thr, and Cys. In particular, the polypeptide practically comprises at least one amino acid residue or peptide residue selected from the group consisting of Gly, Sar, Ser, Glu, Asp, Lys, Arg, Pro, and Val. Specifically, for example, the polypeptide preferably comprises the amino acid residue or peptide residue such as Gly, Sar, Ser, Glu, Asp, Lys, Arg-Gly-Asp, Tyr-Ile-Gly-Ser-Arg, Ile-Lys-Val-Ala-Val, Val-Pro-Gly-Val-Gly, Asp-Gly-Glu-Ala, Gly-Ile-Ala-Gly, His-Ala-Val, Glu-Arg-Leu-Glu, Lys-Asp-Pro-Lys-Arg-Leu, or Arg-Ser-Arg-Lys.

The polypeptide of the present invention may be a physiologically or pharmacologically acceptable salt. For example, the polypeptide may form a salt with a salt-forming (or salt-formable) compound such as an inorganic acid (e.g., hydrochloric acid, sulfuric acid, and phosphoric acid), an organic acid (e.g., acetic acid, trifluoroacetic acid, lactic acid, tartaric acid, maleic acid, fumaric acid, oxalic acid, malic acid, citric acid, oleic acid, and palmitic acid), a metal (e.g., an alkali metal such as sodium or potassium, an alkaline earth metal such as calcium, and aluminum), an organic base (e.g., trimethylamine, triethylamine, t-butylamine, benzylamine, diethanolamine, dicyclohexylamine, and arginine). These salt-forming compounds maybe used singly or in combination. These salts maybe obtained by a usual salt-forming reaction.

In the polypeptide of the present invention, the proportion (molar ratio) of the peptide unit (1) relative to the peptide unit (2) [(1)/(2)] is about 99/1 to 1/99, preferably about 98/2 to 2/98, and more preferably about 95/5 to 5/95.

The proportion (molar ratio) of the total amount of the peptide units (1) and (2) relative to other peptide unit [the former/the latter] is about 100/0 to 50/50, preferably about 100/0 to 60/40, and more preferably about 100/0 to 70/30.

Such a polypeptide forms a chain (or linear) polypeptide without forming a ring such as a six-membered ring by cyclization and is soluble or partially soluble in a solvent (a hydrophilic solvent, e. g. , water, a sulfoxide such as dimethyl sulfoxide, dimethylformamide, dimethylacetamide, and N-methylpyrrolidone, or a mixed solvent thereof). The polypeptide of the present invention shows, for example, a peak corresponding to the molecular weight in a range from about 5 x 10³ to 500 x 10⁴, preferably from about 1 x 10⁴ to 300 x 10⁴, and preferably about 3 x 10⁴ to 200 x 10⁴, and more preferably 5 x 10⁴ to 100 x 10⁴ in terms of a globular protein by means of an aqueous gel permeation chromatography (GPC).

Further, the polypeptide of the present invention shows positive Cotton effect at a wavelength in range of 220 to 230 nm and negative Cotton effect at a wavelength in range of 195 to 205 nm in circular dichroism spectra. At least one part (that is, part or all) of the polypeptide is, accordingly, capable of forming a triple helical structure, and the polypeptide forms a collagenous (collagen-like) polypeptide. Incidentally, Cotton effect means a phenomenon caused by difference between an absorption coefficient relative to a right circularly polarized light and that relative to a left at a specific wavelength in an optical rotatory substance.

The polypeptide of the present invention is capable of forming a collagen-like fiber assembly structure. The polypeptide chain having the above-mentioned triple helical structure can self-assemble to form a fibril-like structure having a length of several nanometers to several tens nanometers. Further, these fibrils can be arranged to form a fiber structure having a length of several micrometers to several tens micrometers. These structures can be observed by a transmission electron microscope, a scanning electron microscope, or an atomic force microscope.

The polypeptide (particularly, the fiber assembly) is capable of allowing an apatite (e.g., a hydroxyapatite) to precipitate or deposit thereon under a body fluid or an environment analogous to a body fluid (or an environment analogous to a living body).

The polypeptide may have an apatite supported thereon. Such a polypeptide (a complex or composite of a polypeptide and an apatite) is also included in the present invention. Hereinafter, such a complex or composite is sometimes simply referred to as a complex.

The representative compounds of the apatites include a hydroxyapatite (Ca₁₀(PO₄)₆(OH)₂). The apatites alsoincludeacalciumphosphate-basedcompound, for example, calcium hydrogenphosphate (CaHPO₄) and calcium phosphate (Ca₃(PO₄)₂).

The amount of the apatites to be supported may be selected from a wide range as usage. For example, the amount of the apatites relative to 100 parts by weight of the polypeptide may be selected from the range of about 0 to 5000 parts by weight and may be preferably about 0.1 to 3000 parts by weight and more preferably about 1 to 1000 parts by weight.

The polypeptide or the polypeptide of the complex, if necessary, may be crosslinked with a crosslinking agent. The crosslinking agent may include, for example, a physiologically acceptable crosslinking agent such as a dialdehyde (e.g., glyoxal, glutaraldehyde, and succinaldehyde), dextran dialdehyde, or aldehyde starch. The proportion of the crosslinking agent relative to 100 parts by weight of the polypeptide may be about 1 to 20 parts by weight and preferably 1 to 10 parts by weight.

Since the polypeptide (including the complex) of the present invention is chemically synthesized from pure reagent(s), the polypeptide is free from a risk of an infection of a pathogenic organism or a transmission of a causative factor [e.g., a protein converted into a pathological protein (such as abnormal prion)] or an undesirable side effect. Therefore, the polypeptide of the present invention is highly safe. Moreover, the polypeptide is excellent in cytophilic property and biocompatibility. The polypeptide is, therefore, useful as a biomaterial or a biocompatible material, for example, an artificial collagen. Further, the polypeptide has a high moisturizing property and a high stability and is useful for a component of a cosmetic preparation, a food composition (a food, as well as an animal feeding stuff for domestic animals, pets, fish, and others).

The polypeptide is, for example, effectively utilizable for supplementing, repairing, and/or regenerating a defective part of a hard tissue (e.g., a born and a cartilage). In such an application, the polypeptide may be used, for example, in the form of a fiber assembly directly or in the form of a complex of the polypeptide (fiber assembly) and an apatite formed in advance under an environment analogous to a body fluid.

The polypeptide (also including the complex) of the present invention may be applied to a variety of tissues (e.g., a born and a cartilage) of a subject. The subject may include human beings and nonhuman animals (nonhuman animals such as monkeys, sheep, bovines, horses, dogs, cats, rabbits, rats, and mice).

In the present invention, the form to be used of the polypeptide (including the complex) is not particularly limited to a specific one. For example, the polypeptide may be in a liquid form (e.g., a solution or a suspension), a particulate form, a one-dimensional substrate (e.g., a fiber or a filiform substrate, a linear substrate, and a rod substrate), a two-dimensional substrate (e.g., a film (or a sheet) or a plate substrate), a three-dimensional substrate (e.g., a tube substrate), and others. Further, the solid polypeptide (including the complex) may be either a non-porous body or a porous body (forexample, a particulate porous body, a two-dimensional porous body such as a non-woven fabric or a woven fabric, and a three-dimensional porous body having a cylindrical form).

The polypeptide (including the complex) may be formed into a desired shape (or form) by molding through a conventional manner as usage. For example, a sheet or film of the polypeptide (or the complex) may be obtained by casting a solution or suspension of the polypeptide (or the complex) on a releasable substrate (support) (e.g., a sheet made from a fluorine-containing resin (a polytetrafluoroethylene)), drying the resulting coat, and separating the coat from the substrate. Moreover, a fiber (or fibrous) substance may be obtained by forcing or injecting a solution or suspension of the polypeptide (or the complex) through a nozzle into a poor solvent to the polypeptide or the complex (or a solution containing a salt of high concentration) . Further, a gelatinous or gel-like substance may be obtained by allowing to stand an aqueous solution or suspension of the polypeptide (or the complex), or if necessary, with adding a polyvalent crosslinking reagent (e.g., glutaraldehyde) thereto. Further, a sponge-like porous substance may be obtained by lyophilizing the resultant gelatinous substance. Furthermore, a porous substance can be also obtained by beating (or foaming) an aqueous solution or suspension of the polypeptide (or the complex) with stirring or other means and drying the resulting matter.

In addition, the polypeptide (including the complex) may be used as a coating agent. For example, a surface of a substrate may be coated with the polypeptide (or the complex) by coating or spraying a solution or suspension of the polypeptide (or the complex) on the surface of the substrate and drying the coated or sprayed layer. The substrate may include a molded (or shaped) product made of various materials such as a metal, a ceramic, and a polymer (such as a synthetic polymer or a natural polymer). Incidentally, a substrate such as a polymeric molded product may have biodegradability or degradability in a living body.

The form of the molded product is not particularly limited to a specific one. The molded product may be in a particulate form, a one-dimensional substrate (e.g., a fiber or a filiform substrate, a linear substrate, and a rod-like substrate), a two-dimensional substrate (e.g., a film (or a sheet) or a plate-like substrate), a three-dimensional substrate (e.g., a tubular substrate), and others. Further, the substrate may be either a non-porous body or a porous body (for example, a particulate porous body, a cellulose fiber paper, a two-dimensional porous body such as a non-woven fabric or a woven fabric, and a three-dimensional porous body having a cylindrical form). Such a porous substrate may be impregnated with a solution or suspension of the polypeptide or the complex to hold the polypeptide or the complex of the polypeptide and an apatite formed thereon. Incidentally, if necessary, the substrate may be surface-treated with a finishing (or surface-treating) agent (e.g., a physiologically acceptable finishing agent).

If necessary, the polypeptide (including the complex) may be sterilized or pasteurized. In particular, the polypeptide or the complex is usually sterilized or pasteurized in a medical purpose in practical cases. A sterilization or pasteurization method may include a conventional method, for example, pasteurization with steam such as a heated and damp steam, pasteurization with a gamma ray, pasteurization with ethylene oxide gas, sterilization with a pharmaceutical preparation, sterilization with an ultraviolet ray, and others. Among these methods, pasteurization with a gamma ray and pasteurization with ethylene oxide gas are preferred from the viewpoint of pasteurization efficiency and low (or small) adverse effects on a material to be used.

### (Process for producing polypeptide)

The novel polypeptide of the present invention may be obtained by a conventional process which comprises subjecting an amino acid or a peptide fragment (or segment) to a condensation reaction, and is not particularly limited to a specific one as long as the polypeptide contains the peptide unit (1) and the peptide unit (2). For example, the polypeptide may be obtained by utilizing a condensation reaction between constituent amino acids, or a condensation reaction between a peptide fragment and an amino acid. These polypeptides are preferably obtained by a process which comprises preparing a peptide having amino acid sequence (s) represented by the formula (1) and/or the formula (2) in advance and condensing a peptide component containing the peptide.

In the process which comprises condensing the peptide component prepared in advance, the peptide chain of the peptide component can be synthesized in accordance with a conventional peptide synthesis method. Peptides may, for example, be prepared based on a solid-phase synthesis method or a liquid-phase synthesis method, and the solid-phase synthesis method is operationally convenient [for example, see "Zoku Seikagaku Jikken Kouza 2, Tanpakushitsu no Kagaku (Supplemental Handbook of Biochemical Experiments, Chemistry of Protein) (the second volume) " edited by The Japanese Biochemical Society (issued by Tokyo Kagaku Dozin Co., Ltd., May 20, 1987), pp. 641 to 694]. For the peptide synthesis, a conventional manner may be utilized, and the manner may include, for example, a coupling method using a condensing agent, an active esterification method (e.g., a phenyl ester such as p-nitrophenyl ester (ONp) and pentafluorophenyl ester (Opfp), an N-hydroxydicarboxylic imide ester such as N-hydroxysuccinimide ester (ONSu), and 1-hydroxybenzotriazole ester(Obt)),a mixed acid anhydride method, an azide method, and others. In the preferredmanner, at least a condensing agent (preferably a condensing agent as mentioned below, in particular a combination of a condensing agent as mentioned below with a condensing auxiliary as mentioned below) may be practically used.

Furthermore, in the peptide synthesis, protection of an amino group, a carboxyl group, and other functional groups (e.g., a guanidino group, an imidazolyl group, a mercapto group, a hydroxyl group, and an ω-carboxyl group) with a protective group, and elimination or removal of the protective group with a catalytic reduction or a strong acid treatment (e.g., anhydrous hydrogen fluoride, trifluoromethanesulfonic acid, and trifluoroacetic acid) are repeatedly conducted depending on a species of amino acids or peptide segments. For example, as a protective group for an amino group, there may be utilized benzyloxycarbonyl group (Z), p-methoxybenzyloxycarbonyl group (Z(OMe)), 9-fluorenylmethoxycarbonyl group (Fmoc), t-butoxycarbonyl group (Boc), 3-nitro-2-pyridinesulfenyl group (Npys), and the other groups. As a protective group for a carboxyl group, there may be utilized benzyloxy group (OBzl), phenacyloxy group (OPac), t-butoxy group (OBu), methoxy group (OMe), ethoxy group (OEt), and the other groups. Incidentally, an automatic synthesis apparatus may be utilized for the peptide synthesis.

More specifically, the preparation of the peptide chain with the solid-phase synthesis method may be carried out in accordance with a conventional manner. As a solid-phase resin (or a carrier), there may be utilized a polymer insoluble to a reaction solvent, for example, a styrene-divinylbenzene copolymer (e.g., a chloromethyl resin, a hydroxymethyl resin, a hydroxymethylphenylacetamidemethyl resin, and a 4-methylbenzhydrylamine resin).

In the solid-phase synthesis method, a peptide can be usually produced by the following steps: a step forming a peptide chain corresponding to an objective peptide, which comprises operations (i) to (iii) mentioned below, and a step comprising (iv) detaching the peptide chain from the polymer (resin) and eliminating the protective group(s) from the protected functional group(s) to obtain the objective peptide, and purifying the resulting peptide. The peptide chain-forming step comprises (i) bonding an amino acid or peptide fragment to the above polymer (resin) from C-terminal to N-terminal of the objective peptide, in which the amino acid or peptide fragment has a free α-COOH group and a functional group (s) (e.g., at least an α-amino group of the N-terminal) protected with a protective group(s), (ii) eliminating the protective group from the α-amino group for forming a peptide bond among the bonded amino acid or peptide fragment, and (iii) sequentially repeating the above bonding operation and the eliminating operation to elongate the peptide chain for the formation of the object peptide. In the operation (i) for bonding the amino acid or peptide fragment, an amino acid which is corresponding to the C-terminal of the peptide chain and has a free α-COOH group, and in which at least the N-terminal is protected with a protective group (for example, an Fmoc-amino acid, a Boc-amino acid) is used. Incidentally, from the viewpoint of inhibiting a side reaction, detachment of the peptide chain from the polymer is preferably carried out concurrently with elimination of the protective group with the use of trifluoroacetic acid. Moreover, the resulting peptide may be purified by utilizing a separation and purification means (e.g., a reversed phase liquid chromatography, and a gel-permeation chromatography).

A process for condensing a peptide component containing thus synthesized peptide may include (a) a process which comprises condensing a peptide component at least containing a peptide having the both amino acid sequences represented by the formulae (1) and (2); and (b) a process which comprises condensing a peptide component at least containing a peptide having an amino acid sequence represented by the formula (1) and a peptide having an amino acid sequence represented by the formula (2).

In the former process (a), the peptide having the both amino acid sequences represented by the formulae (1) and (2) (that is, a peptide having both a peptide unit having an amino acid sequence represented by the formula (1) and a peptide unit having an amino acid sequence represented by the formula (2)) may be used singly or in combination. Moreover, in this process, as a peptide component, other peptide(s) may be used in addition to the above-mentioned peptide having the both units, depending on an object polypeptide. Other peptide(s) may include, for example, a peptide containing the above-mentioned other amino acid residue (s) or peptide residue (s), and others. These other peptides maybe used singly or in combination. Incidentally, in the process, the proportion of the unit (1) or (2) may be easily adjusted by co-condensing a peptide having an amino acid sequence represented by the formula (1) or (2).

Also, in the latter process (b), (1) a peptide (oligo- or polypeptide unit) having an amino acid sequence represented by the formula (1), and (2) a peptide having an amino acid sequence represented by the formula (2) may be used singly or in combination, respectively. Moreover, also in the process, as a peptide component, other peptide (s) may be used in addition to these peptides (1) and (2), depending on an object polypeptide. Examples of other peptide(s) may include a peptide containing the above-mentioned other amino acid residue(s) or peptide residue (s), and others. These other peptides may be used singly or in combination.

The condensation reaction of these peptides is usually carried out in a solvent. The solvent may be capable of dissolving or suspending (partly or wholly dissolving) the peptide components and compounds, and there may be usually employed water and/or an organic solvent. Examples of the solvent may include water, an amide (e.g., dimethylformamide, dimethylacetamide, and hexamethylphosphoramide), a sulfoxide (e.g., dimethyl sulfoxide), a nitrogen-containing cyclic compound (e.g., N-methylpyrrolidone and pyridine), a nitrile (e.g., acetonitrile), an ether (e.g., dioxane and tetrahydrofuran), an alcohol (e.g., methyl alcohol, ethyl alcohol, and propyl alcohol), and a mixed solvent thereof. Among these solvents, water, dimethylformamide, or dimethyl sulfoxide is practically used.

The reaction of these peptides may be usually carried out in the presence of at least a dehydrating agent or a condensing agent (or a dehydrating and condensing agent) . The reaction with these peptide components in the presence of a dehydrating and condensing agent and a condensing auxiliary (synergist) smoothly produces a polypeptide with inhibiting dimerization or cyclization.

The dehydrating and condensing agent is not particularly limited to a specific one as far as the agent can conduct dehydration and condensation efficiently in the above-mentioned solvent. For example, the dehydrating and condensing agent (the dehydrating agent) may include a carbodiimide-series condensing agent [e.g., diisopropylcarbodiimide (DIPC), 1-ethyl-3-(3-dimethylaminopropyl)-carbodiimide (EDC=WSCI), 1-ethyl-3-(3-dimethylaminopropyl)-carbodiimide hydrochloride (WSCI·HCl), and dicyclohexylcarbodiimide (DCC)], a fluorophosphate-series condensing agent [e.g., O-(7-azabenzotriazol-1-yl)-1,1,3,3-tetramethyluronium hexafluorophosphate, O-benzotriazol-1-yl-N,N,N',N'-tetramethyluronium hexafluorophosphate, benzotriazol-1-yl-oxy-tris-pyrrolidinophosphonium hexafluorophosphate, and a salt of benzotriazol-1-yl-tris(dimethylamino)phosphonium hexafluorophosphide (BOP)], diphenylphosphorylazide (DPPA), and others. The dehydrating and condensing agent (s) maybe used singly or used as a mixture in combination thereof. The preferred dehydrating and condensing agent includes a carbodiimide-series condensing agent [e.g., 1-ethyl-3-(3-dimethylaminopropyl)-carbodiimide, and 1-ethyl-3-(3-dimethylaminopropyl)-carbodiimide hydrochloride].

The condensing auxiliary is not particularly limited to a specific one as long as the condensing auxiliary can facilitate the reaction of the condensing agent. For example, there may be mentioned an N-hydroxypolycarboxylic imide [e.g., an N-hydroxydicarboxylic imide such as N-hydroxysuccinic imide (HONSu) or N-hydroxy-5-norbornene-2,3-dicarboxylicimide (HONB)]; an N-hydroxytriazole [e.g., an N-hydroxybenzotriazole such as 1-hydroxybenzotriazole (HOBt)]; a triazine such as 3-hydroxy-4-oxo-3,4-dihydro-1,2,3-benzotriazine (HOObt) ; ethyl ester of 2-hydroxyimino-2-cyanoacetic acid; and others. These condensing auxiliaries may be also used singly or in combination. The preferred condensing auxiliary includes an N-hydroxydicarboxylic imide [e.g., HONSu], an N-hydroxybenzotriazole or N-hydroxybenzotriazine [e.g., HOBt].

The dehydrating and condensing agent maybe suitably used in combination with the condensing auxiliary. As a combination of the dehydrating and condensing agent with the condensing auxiliary, there may be mentioned, for example, DCC-HONSu (HOBt or HOObt), WSCI-HONSu (HOBt or HOObt), and other combinations.

The amount to be used of the dehydrating and condensing agent is, in a water-free solvent system, usually about 0.7 to 5 mol, preferably about 0.8 to 2.5 mol, and more preferably about 0.9 to 2.3 mol (e.g., about 1 to 2 mol) relative to 1 mol of the total molar amount of the peptides. In a water-containing solvent (or an aqueous solvent) system, since the dehydrating and condensing agent may be deactivated by water, the amount to be used of the dehydrating and condensing agent is usually about 2 to 500 mol (e.g., about 2 to 50 mol), preferably about 5 to 250 mol (e.g., about 5 to 25 mol), and more preferably about 10 to 125 mol (e.g., about 10 to 20 mol) relative to 1 mol of a total molar amount of the peptides. The amount to be used of the condensing auxiliary is, for example, about 0.5 to 5 mol, preferably about 0.7 to 2 mol, and more preferably about 0.8 to 1.5 mol relative to 1 mol of a total molar amount of the peptides irrespective of a kind or species of the solvent.

In the condensation reaction in the present invention, the pH of the reaction system may be adjusted, or a base being inert for the reaction may be added to the system. The pH may be usually adjusted with an inorganic base [e.g., sodium hydroxide, potassium hydroxide, sodium carbonate, and sodium hydrogencarbonate], an organic base, an inorganic acid (e.g., hydrochloric acid), or an organic acid. The pH of the reaction mixture is usually adjusted to approximately neutral pH (pH = about 6 to 8). As the base being inert for the reaction, there may be exemplified a tertiary amine (e.g., a trialkylamine such as trimethylamine, triethylamine, or diisopropylethylamine, and a heterocyclic tertiary amine such as N-methylmorpholine or pyridine), and others. The amount of the base may be usually selected within a range from one to two times as much as the total molar amount of the peptides.

The formation of a triple helical structure in the polypeptide of the present invention can be usually proved by measuring circular dichroism spectra for a solution of the polypeptide. In particular, regarding circular dichroism spectra, it has been reported that a naturally-occurring collagen and peptide chain forming a triple helical structure distinctively shows positive Cotton effect at a wavelength in range of 220 to 230 nm and negative Cotton effect at a wavelength in range of 195 to 205 nm (J. Mol. Biol., Vol. 63 pp. 85 to 99, 1972).

In the present invention, by bringing the polypeptide into contact with an aqueous solution containing a calcium ion and a phosphate ion (or a simulated body fluid or a pseudo-body fluid), the polypeptide (particularly the fiber assembly) may have an apatite supported (precipitated or deposited) thereon to form a complex of the polypeptide and the apatite.

In such a process, calcium ion may be introduced into the aqueous solution by using various calcium compounds. The calcium compound may include, for example, a calcium halide (e.g., calcium chloride), calcium hydroxide, calcium oxide, a salt of an inorganic acid (e.g., calcium nitrate, calcium sulfate, calcium carbonate, and calcium hydrogencarbonate), and a salt of an organic acid (e.g., calcium acetate). These calcium compounds may be used singly or in combination.

In addition, phosphate ion may be also introduced into the aqueous solution by using various phosphoric acid components. The phosphoric acid component may include, for example, orthophosphoric acid or a salt thereof [e.g., an alkali metal phosphate such as sodium phosphate or potassium phosphate; an alkali metal hydrogenphosphate such as sodium hydrogenphosphate or potassium hydrogenphosphate; an alkaline earth metal hydrogenphosphate such as a calcium hydrogenphosphate (e.g., sodium dihydrogenphosphate); an ammonium salt such as ammonium phosphate, ammonium hydrogenphosphate, or ammonium sodium hydrogenphosphate]. These phosphoric acid components may be used singly or in combination.

The concentrations of the calcium ion and the phosphate ion in the aqueous solution are not particularly limited to a specific one as long as the efficiency in the apatite supporting (precipitating or depositing on the polypeptide) is not deteriorated. According to the present invention, the apatite can be supported to the polypeptide efficiently even in an aqueous solution containing calcium ion and phosphate ion in low concentrations. The aqueous solution preferably contains calcium ion and phosphate ion that are supersaturated to calcium phosphate (that is, the solution preferably contains calcium ion and phosphate ion at concentrations in excess of the solubility of calcium phosphate). Incidentally, it is not preferred to precipitate calcium phosphate in the aqueous solution. However, calcium phosphate may be precipitated as long as the aqueous phase (or the water phase) is in supersaturation.

For example, at a temperature of 36.5°C, the concentration of calcium ion in the aqueous solution may be selected from the range of about 2 to 50 mM (mmol/L) and usually not lower than 2.5 mM (e.g., about 2.5 to 30 mM), preferably about 2.5 to 20 mM, and more preferably about 3 to 10 mM. Moreover, at a temperature of 36.5°C, the concentration of the phosphate ion in the aqueous solution may be selected from the range of about 0.5 to 50 mM (mmol/L) and may be usually about not lower than 1 mM (e.g., about 1 to 30 mM), preferably about 1 to 20 mM, and more preferably about 1.2 to 10 mM.

Incidentally, the aqueous solution may contain other ionic species, for example, a metal ion [for example, an alkali metal ion (e.g., a sodium ion and a potassium ion), an alkaline earth metal ion (e.g., a magnesium ion), a transition metal ion (e.g., a titanium ion, a zirconium ion, and a cobalt ion), and a silicon ion], a halogen ion (e.g., a bromine anion, a chlorine anion, and a fluorine anion), and an ion of an inorganic acid (e.g., a carbonate ion, a bicarbonate (or a hydrogencarbonate) ion, and a sulfate ion) . These ionic species may be selected singly or in combination in correspondence to the formation (or composition) or the crystal structure of the calcium phosphate-based compound.

The pH of the aqueous solution containing calcium ion and phosphate ion may be adjusted with a buffer. As the buffer, a Tris (tris(hydroxymethyl)aminomethane) buffer, aphosphoricacid-seriesbuffer, aboricacid-series buffer,a carbonic acid-series buffer,a citric acid-series buffer, an acetic acid-series buffer, and others may be utilized. The pH of the aqueous solution is usually about 6 to 8.

As the representative aqueous solutions, solutions having the following formations may be exemplified:
(1) an aqueous solution that contains Na⁺ (142 mM), K⁺ (5 mM), Ca²⁺ (2.5 mM), Mg²⁺ (1.5 mM), Cl⁻ (148.8 mM), HCO₃⁻ (4.2 mM), HPO₄²⁻ (1 mM), and SO₄²⁻ (0.5 mM) and has a pH of 7.4 adjusted with a Tris buffer, and
(2) an aqueous solution that contains Na⁺ (213 mM), K⁺ (7.5 mM), Ca²⁺ (3.8mM), Mg²⁺ (2.3 mM), Cl⁻ (223.3 mM), HCO₃⁻ (6.3 mM), HPO₄²⁻ (1.5 mM), and SO₄²⁻ (0.75 mM) and has a pH of 7.4 adjusted with a Tris buffer.

The process for bringing the polypeptide of the present invention into contact with the aqueous solution may be any process as long as the polypeptide can be coated with the apatite (a calcium phosphate-based compound). The process may be a spraying process, an impregnation process, or a coating process. The convenient one includes a process of bringing the polypeptide into contact with the apatite in an aqueous solution.

The contact temperature of the polypeptide and the aqueous solution (e.g., the impregnation temperature) may be suitably selected from the range of 10 to 100°C depending on the species of the apatite (the calcium phosphate-based compound) to be coated. The contact temperature is usually about 10 to 60°C, preferably about 20 to 50°C, and more preferably about 25 to 45°C (e.g., about 30 to 39°C) . The contact time (e.g., the impregnation time) may be selected depending on the kinds of the apatite and the objective precipitated amount of the apatite. The contact time is usually within 10 days (particularly within 7 days). Incidentally, in the contact of the polypeptide in the aqueous solution, the solution is usually stirred slowly or may be allowed to stand to form an apatite.

### [Polypeptide-containing composition]

It is sufficient that the polypeptide-containing composition of the present invention contains at least the above-mentioned polypeptide (or the above-mentioned complex). The application of such a composition is not particularly limited to a specific one. In particular, the composition is useful for a cosmetic preparation, a food composition, and others.

In many cases such a composition may usually comprise abase material (or carrier), an effective (active) ingredient (e.g., in the application of the cosmetic preparation, a moisturizer), and an additive (e.g., in the food composition, a food additive, a flavor enhancer or seasoning), and others. The composition may contain the polypeptide (or the complex) as at least one component among these components. Moreover, the form (or shape) of the composition is not particularly limited to a specific one and may be in the form of a solid, a semisolid, or a liquid.

The content of the polypeptide may be selected from the wide range depending on the kinds or form of the composition, for example, about 0.001 to 99% by weight. In the use of the polypeptide as a base material, the proportion of the polypeptide may be, for example, about 10 to 99% by weight (e.g., about 10 to 90% by weight), preferably about 20 to 97% by weight (e.g., about 20 to 80% by weight), and more preferably about 30 to 95% by weight (e.g., about 30 to 70% by weight), relative to the whole composition (such as the cosmetic preparation or the food composition). In the use of the polypeptide as an effective ingredient, the proportion may be, for example, about 0.001 to 95% by weight (e.g., about 0.001 to 90% by weight), preferably about 0.01 to 90% by weight (e.g., about 0.01 to 80% by weight), and more preferably about 0.1 to 90% by weight (e.g., about 0.1 to 70% by weight). Moreover, in the use of the polypeptide as an additive, the proportion may be about 0.001 to 40% by weight, preferably about 0.01 to 30% by weight, and more preferably about 0.1 to 20% by weight.

In the composition, the polypeptide may be used in combination with other base material (or carrier). Such a base material may be suitably selected depending on the form of the composition and include a solid base material (e.g., a powdery base material), a semisolid base material (e.g., a gel base material, and an ointment or plaster base material), a liquid base material, and others. These other base material may be either an aqueous base material or an oily base material.

Among the above-mentioned other base materials, the powdery base material may include a saccharide (e.g., a monosaccharide or a polysaccharide such as glucose, lactose, or a starch; and a sugar alcohol such as sorbitol), an amino acid (e.g., serine, glycin, threonine, and alanine), and others. Moreover, in the application of the cosmetic preparation, as the powdery base material, there may be used a metal soap (e.g., a metal salt of a fatty acid, for example, potassium stearate, a sodium salt of a palm-oil fatty acid, magnesium myristate, and calcium stearate), a resin [for example, a thermoplastic resin such as an olefinic resin such as a polyethylene, a styrenic resin, an acrylic resin, a vinyl alcohol-series polymer, a vinyl carboxylate-series resin, a polyamide-series resin, or a polyester-series resin; and a thermosetting resin such as a phenolic resin, an amine resin (e.g., a urea resin, and a melamine resin), a thermosetting acrylic resin, an unsaturated polyester resin, an alkyd resin, an epoxy resin, or a silicone resin (e.g., a poly(methylsiloxane))], an inorganic powder component [e.g., a sericite, an extender (e.g., a natural clay mineral such as a kaolin, a talc, or a mica; a synthetic fluorphlogopite, and a hexagonal boron nitride)]. Further, in the food composition, as the powdery base material, there may be used a protein (e.g., a protein such as a soy protein), a polyvinylpyrrolidone, and others.

The solid or semisolid base material may include a solid or semisolid oily base material derived from plants and animals (e.g., a bees wax, a Japan tallow (or Japanese wax), a carnauba wax, a candelila wax, a cacao butter, and a beef tallow (or a beef dripping); and a lanolin), a solid or semisolid oily base material derived from a mineral (e.g., a solid paraffin, a ceresin, a microcrystalline wax; and a vaseline), and in addition, a fatty acid ester (e.g., an alkyl ester of a saturated or unsaturated fatty acid such as cetyl 2-ethylhexanoate; an alkyl ester of a saturated or unsaturated hydroxylic acid such as isostearyl malate; and an ester of a saturated fatty acid with a polyhydric alcohol such as glyceryl monostearate or ethylene glycol distearate), a higher alcohol (e.g., a saturated aliphatic alcohol such as cetyl alcohol, stearyl alcohol, or oleyl alcohol), a higher fatty acid (e.g., stearic acid, and oleic acid), a gel base material (e.g., a viscous fluid (or substance)), and others. Moreover, for the food composition, as the solid or semisolid oily base material derived from plants and animals, a margarine, a shortening, a butter, and a lard may be also used. The viscous fluid of the gel base material may include an animal- or plant-series viscous fluid (e.g., a gum such as a queenseed gum, a tragacanth gum, or a xanthan gum; a saccharide such as a pectin or a starch; corsican pearlwort; an alginic acid compound such as a sodium alginate or a propylene glycol alginate; a polysaccharide such as a hyaluronic acid, a sodium chondroitin sulfate, or a chondroitin heparin; and a protein such as a casein, a vitronectin, a fibronectin, a keratin, an elastin, a soy protein, or a royal jelly), a cellulose or a derivative thereof (e.g., a cellulose; a methylcellulose, an ethylcellulose, a carboxymethylcellulose, and a hydroxyethylcellulose), a synthetic polymer (e.g., a poly(sodium acrylate), a polyvinyl alcohol, a polyvinyl methyl ether, a polyvinylpyrrolidone, a carboxyvinyl polymer, and a polyoxyalkylene glycol having a highmolecular weight (e.g., a polyethylene glycol)), an inorganic viscous fluid (e.g., "VEEGUM", a bentonite, an organo-modified bentonite, and a swelling bentonite), a gum base (e.g., a chewing gum base such as methyl acetylricinoleate or a vinyl acetate resin), and others.

The liquid base material may include, for example, an oily base material such as an oily base material (e.g., an animal or vegetable oil and fat such as a jojoba oil, an olive oil, a cocoanut oil, a camellia oil, a macadamia nut oil, a castor oil, a soybean oil, a rapeseed oil, a cottonseed oil, a safflower oil, a peanut oil, a corn oil, a sesame oil, a grapeseed oil, or squalane), a mineral-series oily base material (e.g., a liquid paraffin, a polybutene, and a silicone oil), a synthetic oily base material (e.g., a synthetic ester oil, and a synthetic polyether oil); an aqueous base material, for example, water, a water-soluble organic solvent [e.g., a lower aliphatic alcohol (e.g., ethanol, and isopropanol); an alkylene glycol (e.g., a polyoxyalkylene glycol having a low molecular weight, or a monoalkyl ester thereof, such as ethylene glycol, diethylene glycol, propylene glycol, dipropylene glycol, 1, 3-butylene glycol, a polyethylene glycol, or diethylene glycol monoethyl ester); a polyhydric alcohol such as glycerin or pentaerythritol; and a carboxylic acid such as lactic acid or sodium pyrrolidonecarboxylate]. Incidentally, in the food composition, as the oily base material, a soybean oil, a rapeseed oil, a cottonseed oil, a safflower oil, a peanut oil, a corn oil, a sesame oil, a grapeseed oil, an olive oil, a cocoanut oil, or others is practically used. Moreover, as the aqueous base material, for example, water, ethanol; a lower carboxylic acid such as lactic acid or acetic acid; or others is practically used.

These other base materials may be used singly or in combination.

The proportion of other base material described the above relative to the whole composition (such as the cosmetic preparation or the food composition) may be about 10 to 99.999% by weight, preferably about 10 to 99% by weight, and more preferably about 20 to 95% by weight. Moreover, the proportion of the polypeptide relative to 100 parts by weight of the base material may be about 0.001 to 500 parts by weight, preferably about 0.01 to 300 parts by weight, and more preferably about 0.1 to 100 parts by weight (e.g., about 1 to 50 parts by weight).

The polypeptide may be combined with other effective (active) ingredient(s). As such other effective ingredient, a component different from the above-mentioned other base materials may be used. The effective ingredient may include an enzyme (e.g., protease, lipase, collagenase, gelatinase, amylase, and lysozyme), a coenzyme, a vitamin (e.g., vitamin A, vitamin B, vitamin C, vitamin D, vitamin E, and vitamin K), an amino acid [e.g., tryptophan, cysteine, L-aspartic acid or a salt thereof (e.g., potassium L-aspartate, magnesium L-aspartate, and potassium magnesium L-aspartate), and aminoethylsulfonic acid (taurine)], and others. Moreover, in the application of the cosmetic preparation, as the above-mentioned other effective ingredients, there may be used an astringent (e . g. , a hydroxylic acid or a salt thereof, such as citric acid, lactic acid, or tartaric acid; an aluminum compound such as aluminum chloride; a zinc compound such as zinc sulfate or sulfophenoxozinc; a proanthocyanidin; an extract of a tannin-containing plant such as hamamelis or white birch; a tansy extract, a rhubarb extract, and a horse tail extract), an emollient [e.g., an emulsified product in which an oily component (such as a triglyceride oil, a squalan, or an ester oil) is emulsified with a nonionic emulsifier (such as a monoglyceride)], a moisturizer, an emollient (e.g., salicylic acid or a derivative thereof, lactic acid, and urea), an antioxidant (e.g., tocopherol or a derivative thereof; and a polyphenol such as an anthocyanin), an ultraviolet absorbing agent or an ultraviolet-scattering inorganic pigment, a skin-whitening agent (e.g., ascorbic acid or a derivative thereof, cysteine, a placenta extract, arbutin, koj ic acid, Rucinol, ellagic acid, and a chamomile extract), an antiperspirant (e.g., an astringent such as an aluminum compound, a zinc compound, or a tannin), a dry skin inhibitor (e.g., a glycyrrhizic acid salt, andavitamin compound), an antiinflammatory agent (e.g., allantoin, guaiazulene, glycyrrhizic acid or a salt thereof, a glycyrrhetinic acid or a salt thereof, ε-aminocaproic acid, tranexamic acid, ibuprofen, indomethacin, zinc oxide, or a derivative thereof; and a plant extract such as an arnica extract), a germicide or antibacterial agent (e.g., a quaternary ammonium salt such as benzalkonium chloride or distearylmethylammonium chloride; a benzoic acid compound such as benzoic acid, sodium benzoate, or peroxybenzoate; a salicylic acid compound such as salicylic acid or sodium salicylate; trichlorocarbanilide, and triclosan), and a cell activator (e.g., riboflavin, pyridoxine, nicotinic acid, pantothenic acid, α-tocopherol, or a derivative thereof; and a plant extract such as a strawberry geranium (Saxifraga stolonifera) extract).

Examples of the moisturizer may include an alkylene glycol (e.g., a polyalkylene glycol or a monoalkyl ether thereof, such as ethylene glycol, diethylene glycol, propylene glycol, dipropylene glycol, 1, 3-butylene glycol, a polyethylene glycol, or diethylene glycol monoethyl ether), a polyhydric alcohol such as glycerin or pentaerythritol; lactic acid, sodium pyrrolidonecarboxylate; an amino acid (e.g., serine, glycin, threonine, and alanine) ; a saccharide (e.g., a sugar alcohol such as sorbitol; and a polysaccharide such as a hyaluronic acid, a sodium chondroitin sulfate, or a chondroitin heparin); a protein (e.g., a vitronectin, a fibronectin, a keratin, an elastin, and a royal jelly), and others.

The ultraviolet absorbing agent may include a benzophenone-series absorbent such as oxybenzone, oxybenzonesulfonic acid, or sodium hydroxymethoxybenzophenonesulfonate; a cinnamic acid-series absorbent such as octyl methoxycinnamate, methyl diisopropylcinnamate, ethyl diisopropylcinnamate, isopropyl p-methoxycinnamate, or di-p-methoxycinnamic acid mono-2-ethylhexanoic acid glyceryl; a p-aminobenzoic acid-series absorbent such as p-aminobenzoic acid, ethyl p-aminobenzoate, octyl p-aminobenzoate, or octyl p-dimethylaminobenzoate; a salicylic acid-series absorbent such as octyl salicylate; a dibenzoylmethane-series absorbent such as 4-t-butyl-4'-methoxybenzoylmethane; urocanic acid or an ester thereof; β-isopropylfuranone; β-carotene; and others. The ultraviolet-scattering inorganic pigment may include titanium oxide (titanium dioxide), zirconium oxide, zinc oxide, iron oxide, and others. Moreover, the above-mentioned other effectiveingredientmay alsoinclude, as usage, an effective ingredient of a cosmetic preparation for the hair (e.g., a hair conditioner, and a dandruff inhibitor), an effective ingredient of a cosmetic preparation for pigmented spot and fleck (e.g., a tyrosinase activation inhibitor, and a melanin-reducing agent), an effective ingredient of a cosmetic preparation for acne (e.g., a keratin softening agent such as sulfur, an antiphlogistic, a cortical hormone, and an oil secretion inhibitor), and others.

Moreover, in the food composition, the above-mentioned other effective ingredient may include a nutrient (nutritious component), for example, a food or feed (or fodder) material [e.g., a grain, a bean, a material derived from animals and birds (e.g., a meat, a blood, a fell (animal skin), an animal bone, or an egg), a fish (e. g. , a fish food, a fish blood, a fish skin, a fish bone, or a fish roe), a milk (e.g., a cow milk), a shellfishery (including a shell), a vegetable, a forage, a fruit, a sea weed, and an insect (including a chrysalis and a spliced chrysalis)], in addition, a yeast or yeast extract, a microorganism (e.g., an acidophilus), a hormone, a protein or peptide (e.g., a silk protein and a silk peptide), a saccharide [e.g., a mono- or polysaccharide such as glucose, lactose, or fructose; a macromolecular saccharide or a salt thereof (e.g., sodium chondroitin sulfate and hyaluronate sodium); a sugar alcohol (e.g., mannitol, xylitol, and sorbitol)], and others. The effective ingredient may also include a medicinal ingredient (medicinal properties), for example, an antihistaminic agent (component) (e.g., chlorpheniramine, diphenhydramine, and salts of thereof (e.g., diphenhydramine hydrochloride), an antiallergic agent (component) (e.g., cromoglycic acid, amlexanox, or salts thereof (e.g., sodium cromoglycate)), a stomachic component, a digestive component (gastric digestant component), an antibacterial or pesticidal component (e.g., sulfoneamide such as sulfamethoxazole, or a salt thereof; a quaternary ammonium or a salt thereof (e.g., benzalkonium chloride and benzethonium chloride); and ofloxacin), an acid component (e.g., acetic acid, a black vinegar, or an apple cider vinegar), a galenical component (e.g., a herbal medicinal component such as turmeric or ginseng), and others. Incidentally, the food or feed material may be employed as processed goods (artifact), for example, a crushed material (e.g., a pulverized material such as a paste or a dry powder), an expressed liquid, a pressed material, an extract, a fermented material, and others.

The above-mentioned other effective ingredient may be used singly or in combination.

The proportion of the above-mentioned other effective ingredient relative to the whole composition (such as the cosmetic preparation or the food composition) may be about 0.001 to 99.9% by weight, preferably about 0.01 to 95% by weight (e.g., about 0.01 to 80% by weight), and more preferably about 0.1 to 90% by weight (e.g., about 0.1 to 60% by weight).

Incidentally, in the use of the polypeptide as a moisturizer, the proportion (molar ratio) of the polypeptide relative to other moisturizer (the above-mentioned moisturizer) [the polypeptide/other moisturizer] may be about 0.1/99.9 to 100/0, preferably about 1/99 to 90/10, and more preferably about 5/95 to 80/20.

The polypeptide may be used in combination with other additives. As such other additives, there may be used a component different from the above-mentioned other base materials and other effective ingredients, or others. The additive may include, for example, a surfactant, an inorganic salt (e.g., sodium sulfate, sodium hydrogencarbonate, and potassium chloride), a coloring agent, a caking additive (a viscous fluid mentioned above, for example, a carboxymethylcellulose, a carboxymethylcellulose sodium, and a carrageenan), an opacifying agent, a perfume material (e.g., a synthetic perfume, an essential oil, and an essential oil component), a sweetening agent (e.g., saccharin sodium), an antioxidant (e.g., erythorbic acid, ascorbic acid, guaiac, dibutylhydroxytoluene, α-tocopherol, and sulfite salt), a sweetener (e.g., a starch sugar such as sucrose (cane sugar), D-xylose, or D-sorbit, a beet sugar, an oligosaccharide, ahoney, orastarchsyrup; glycylrrhizine; and stevioside), a plant extract, an emulsifier or a suspending agent [e.g., a surfactant; a polysaccharide such as sodium chondroitin sulfate; a water-soluble polymer, for example, a polyvinylpyrrolidone, a water-soluble cellulose ether (e.g., a methylcellulose, a hydroxyethylcellulose, a hydroxypropylcellulose, and a sodium carboxymethylcellulose); and a soy phospholipid], an enzyme, a preservative (e.g., benzoic acid, sorbic acid, or salts thereof), a pH adjuster (e.g. , a base such as sodium hydrogencarbonate; an acid such as sodium monohydrogen phosphate; and a borax), a chelating agent (e.g., a hydroxycarboxylic acid such as citric acid, and a phosphoric acid such as metaphosphoric acid), a metal ion sequestering agent (e.g., a polyphosphate, ethylenediaminetetraacetate, and phytic acid), a solidifying agent (e.g., the higher alcohols, the saturated fatty acids and the waxes which are exemplified in the paragraph of the base material), a solubilizing agent (e.g., a polyoxyethylene hydrogenated castor oil), a plasticizer [e.g., camphor, a phthalic acid ester (e.g., dibutyl phthalate), and an aliphatic polybasic acid ester such as tributyl acetylcitrate], a gelatinizing agent (e.g., an organo-modified bentonite), a thickening agent (e. g. , the viscous fluids exemplified in the paragraph of the above-mentioned base material), an organic solvent (e.g., an alcohol such as ethanol or butanol), a reducing agent (e.g., thioglycolic acid or a salt thereof, and cysteine), a basic agent or a basic component (e.g., an inorganic base such as ammonia, ammonium carbonate, or calcium carbonate, and an organic base such as ethanolamine), an oxidizing agent (e.g., sodium bromate, hydrogen peroxide, and sodium perborate), an antiseptic or preservative (e.g., paraben, sodium benzoate, a p-hydroxybenzoic acid ester, dehydroacetic acid, and sorbic acid or a salt thereof), an algefacient or refrigerant (e.g., menthol), a buffer (e.g., a buffer solution of a phosphate or a borate), and a dissolution aid (e.g., a polyethylene glycol, ethanol, sodium carbonate, and sodium citrate). Moreover, in the application of the cosmetic preparation, as the above-mentioned other additives, there may be also used, for example, a fiber (e.g., a synthetic fiber such as a nylon fiber, and a natural fiber), an abrasive (or abradant) (e.g., calcium hydrogenphosphate, calcium carbonate, and silicic anhydride), a foaming agent (e.g., sodium lauryl sulfate), and a humectant or wetting agent (e.g., sorbit and glycerin).

In the food composition, as the above-mentioned other additives, there may be also used, for example, conventional food additives or feed additives, for example, a dietary supplement (e.g., a calcium component such as calcium citrate, calcium lactate, or calcium pantothenate; ascorbic acid or a salt thereof or an ester thereof; ferric chloride; a thiamine salt; a nicotine acid compound; and a vitamin), a binder (texturizer) (e.g., pyrophosphate), a thickening agent (e.g., sodium alginate, a propylene glycol alginate, a methylcellulose, a sodium polyacrylate, or a casein), a fermentation regulant (e.g., potassium nitrate, and sodium nitrate), an alkaline chemical (e.g., an alkaline chemical for producing Chinese noodles, such as a kansui, a carbonate, or a phosphate), a sterilizer or germicide (e.g., hydrogen peroxide, hypochlorous acid, or sodium hypochlorite; a bleaching powder), an acidifier or acid component (e.g., acetic acid, citric acid, tartaric acid, malic acid, gluconic acid, succinic acid, or glucono-δ-lactone), a flavor enhancer (seasoning) (e.g., sodium L-aspartate, DL-alanine, glutamic acid, monosodium succinate; a salt (sodium chloride) (table salt), a soy sauce, a fermented bean paste (miso), an alcohol (e.g., a liquor (sake), and a wine, a sweet sake (mirin)), and a flavor enhancer made from fermentation with a salt), an aromatizing agent (e.g., an aroma chemical such as various esters (e.g., ethyl acetoacetate), citral, citronellal, lemon, lime, orange, or strawberry; a refrigerant agent such as peppermint or menthol; or a spice and condiment), an extract agent, a texturizing agent (e.g., D-mannitol), a color-fixing agent (e.g., sodium nitrite, sodium nitrate, and ferrous sulfate), a film-forming agent (e.g., an oxyethylene higher aliphatic alcohol, sodium oleate, and a vinyl acetate resin), a bleaching agent (e.g., sodium hydrogensulfite), an conditioning agent (e.g., L-cysteine monohydrochloride, and calcium stearoyl lactylate), a quality improving agent (e.g., propylene glycol), a solvent (e.g., glycerin), an insecticide (e.g., piperonyl butoxide), a baking powder (e. g., an ammonium alum, an alum, or ammonium chloride), and a release agent (e.g., a liquid paraffin).

Incidentally, among the above-mentioned other additives, the surfactant may include an anionic surfactant (e.g., an alkyl sulfate salt; an alkyl ether sulfate salt such as a sodium alkyl ether sulfate or a triethanolamine alkyl ether sulfate; an acylmethyl taurine salt; an acylglutamate such as sodium acylglutamate; an amide ether sulfate; a sorbitan fatty acid ester such as sorbitan sesquioleic acid ester; a polyoxyethylene sorbitan fatty acid ester; a glycerin fatty acid ester such as glyceryl monostearate; a polyoxyethylene glycerin fatty acid ester such as a polyoxyethylene glyceryl monostearate; a sucrose fatty acid ester; and a propylene glycol fatty acid ester), an ampholytic surfactant (e.g., an alkylacetic acidbetaine, an amidoacetic acid betaine, and an imidazolinium betaine (an amine oxide-based semipolar surfactant)), a nonionic surfactant (e.g., a fatty acid alkanol amide such as lauric acid diethanolamide, or palm fatty acid diethanolamide; a polyoxyethylene alkyl ether such as a polyoxyethylene oleyl ether or a polyoxyethylene octyl dodecyl ether; a polyoxyethylene-polyoxypropylene block copolymer; and a polyoxyethylene hydrogenated castor oil ester), a cationic surfactant (e.g., an alkyltrimethylammonium chloride and a dialkyldimethylammonium chloride), and others.

Incidentally, in the application of the cosmetic preparation, as the coloring agent (dye and pigment), there may be used a synthetic or natural pigment (a dye, a pigment), for example, a tar pigment, an iron oxide-series inorganic pigment, a black iron oxide lake, a white pigment such as titanium dioxide; a pearl pigment (e.g., a titanated mica system, bismuth oxychloride, and argentine); a dye such as Red No. 223, or Orange No. 201; and a natural pigment (e.g., cochineal, and carthamin). Moreover, in the food composition, an edible dye such as β-carotin may be used as the coloring agent.

The above-mentioned other additives may be used singly or in combination.

The proportion of the above-mentioned other additive relative to the whole composition (such as the cosmetic preparation or the food composition) may be about 0.001 to 40% by weight, preferably about 0.01 to 30% by weight, and more preferably about 0.1 to 20% by weight. Incidentally, in the use of the food composition as a food product, the food additives among the additives may be employed in the designated proportion complying with Food Sanitation Law and the like. Moreover, the proportion of additives which do not have a standard to use (e . g. , a natural food additive) may be suitably selected depending on the kinds of food additives or food products.

The above-mentioned base materials, effective ingredients and additives may be used in the form of a salt. Such a salt is preferred to be a physiologically or pharmaceutically acceptable salt, for example, an organic acid salt (e.g., a carboxylic acid salt such as an acetate, a fumarate, or a citrate; and an organic sulfonate such as methanesulfonate), an inorganic acid salt (e.g., a hydrochloride), a salt with an organic base (e.g., a salt with a tertiary amine such as trimethylamine or ethanolamine), a salt with an inorganic base (e.g., an ammonium salt; an alkali metal salt such as a sodium salt; an alkaline earth metal salt such as a calcium salt; and an aluminum salt), and others.

Incidentally, each of the base material, the effective ingredient and the additive may have an interactive property with the polypeptide as long as the properties of the polypeptide are not deteriorated. Preferably, the base material, the effective ingredient and the additive do not usually have the interactive property (e.g., reactivity, and degradability).

Among the above-mentioned compositions, the cosmetic preparation may be utilized as an external composition for applying to a skin. The site to be applied is not particularly limited to a specific one, and may include, for example, integuments of various sites such as head, face, neck, arm, hand, chest protection and foot, and in addition, intraoral parts, body hair such as head hair, eyelash and eyebrow, and nail.

The shape (or form) or configuration of the cosmetic preparation is not particularly limited to a specific one, and may include, for example, a liquid preparation (e.g., a lotion, an emulsion, and a suspension), a semisolid preparation (e.g., a gel, an ointment, a plaster, and a cream), and a solidpreparation (e.g., a powder, and a cake) . The liquid preparation and semisolid preparation may be impregnated or applied to a base or substrate (e.g., a nonwoven fabric, a woven fabric, a paper, and a polymer film), for example, may be used as a facial mask, a mask, a wet tissue, and others. The liquid preparation may be a solution or a dispersion (e.g., a dispersion in which a powder is dispersed in an aqueous liquid preparation, a dispersion of a two-layer liquid preparation composed of water and a nonaqueous organic solvent, and a dispersion in which a powder is dispersed in a two-layer liquid preparation composed of water and a nonaqueous organic solvent) . Moreover, the liquid preparation may be used as a spray or an aerosol. In the spray or the aerosol, the liquid preparation to be sprayed may be in the form of a fog (or mist) or a foam. Incidentally, as an aerosol propellant, a liquefied gas (e.g., a fluorocarbon, a hydrocarbon, a liquefied petroleum gas, and dimethyl ether), a compressed gas (e.g., a compressed inactive gas such as nitrogen gas or carbon dioxide), and others may be used.

The type of usage of the cosmetic preparation may include a basic cosmetic preparation (e.g., a lotion, a skin lotion, a gel lotion, a milky lotion, a cream, and an essence), a makeup cosmetic preparation (e.g., a liquid or powdery foundation, a blusher, an eye shadow, and a hair dressing), a bathwater additive (e.g., a bath agent), and a cleaning agent (e.g., a facial wash, a cleansing agent, a soap, a body shampoo, a shampoo, a rinse, and a conditioner).

Moreover, the cosmetic preparation may also include, depending on the site to be applied or the application (or function), for example, a scalp and hair care cosmetic preparation (e.g., a shampoo, ahairrinse, ahairtreatment, a hair essence, a hair styling agent, a perm agent, a cold wave lotion, and a hair dye), a partial cosmetic preparation [e.g., an eye makeup cosmetic preparation such as an eye liner or a mascara; a lip cosmetic preparation such as a lip balm, a lip essence, a lip rouge, a lip gloss, or a lip makeup remover; an oral cosmetic preparation (e.g., a tooth powder or a toothpaste, a mouthwash, and a mouth refrigerant) ; and a nail cosmetic preparation (e.g., a nail essence, a nail enamel, and an enamel remover)], a tanning or sunburn cosmetic preparation, a cosmetic preparation for pigmented spot and fleck, a cosmetic preparation for acne, and a deodorant cosmetic preparation (e.g., an antiperspirant).

The usage and dose of the cosmetic preparation may be selected depending on the species (application) or configuration (form) of the cosmetic preparation. For example, the cosmetic preparation may be applied to a given site about once to five times a day. For example, the perm agent may be applied to a given site about once to three times a week to per several months. The enamel maybe applied to a given site about once to ten times a day to a week. Moreover, as for usages as the cleaning agent, the hair preparation (e.g., a rinse, a conditioner, a hair treatment, and a perm agent), and others, such a cosmetic preparation may be washed away with water, hot water, or the like after application.

Moreover, among the above-mentioned compositions, the shape (or form) or configuration of the food composition is not particularly limited to a specific one, and may include, for example, a liquid composition, a semisolid composition (e.g., a gel, a cream, a slurry, and a paste), a solid composition (e.g., a powder, a granule, a flake, a cake, a preparation, a gumi, a nougat, a chewable tablet, and a film), and the like. The composition may be in the form of a capsule composition in which contents are encapsulated in a capsule. Moreover, the solid composition may be subjected to a coating treatment such as a sugar coating or an enteric coating.

The food composition may be subjected to, if necessary, a conventional preservative or package treatment such as a freezing treatment (including a freeze drying), a retort treatment, or a canning treatment.

The food composition may be a functional food such as a health food (e.g., a healthy drink such as a nutrition supplement drink), a health supplement (e.g., a nutriceutical food such as various supplements, a food aiming at improvement in eating quality), a food for specified uses [e.g., an invalid food (patient food), and a senior food (food for senior)], a food with health claims (e.g., a nutriceutical functional food, and a specified health food), and others.

Moreover, the food composition may have the same or resembling properties with the naturally occurring collagen, for example, effects such as a furnishing of nutrition, a moisturizing action or an antiaging effect (improvement in moisturizing action inside of mouth or throat, a protection of gastrointestinal mucosa and endosporium, accerlation of producing collagen, accerlation of increasing osteoblast or fibroblast, increase of metabolism, retention of water in corneum of skin, improvement in skin fitness, prevention of wrinkle or improvement thereof, or activation of skin), strengthening of bone (including prevention of osteoporosis or improvement thereof, and improvement in bone density), and others.

### INDUSTRIAL APPLICABILITY

The polypeptide and the complex of the present invention is utilizable for medical materials such as biological implantable materials (e.g., an artificial bone, an artificial tooth root, a bone-repairing agent, and a bone-supplementing agent), carriers or supports for systems engineering (e.g., for a hard tissue), or carriers or supports for regeneration medicines, materials for pharmaceutical preparations (e.g., a base material and an additive), materials for cosmetic preparations (e.g., a base material and an additive), food additives, coating agents, and others. In particular, the composition containing the above-mentioned polypeptide is, for example, useful for various applications of cosmetic preparations, e.g., basic cosmetic preparations, makeup cosmetic preparations, bathwater additives, and cleaning or washing agents. In addition, the composition may be utilized, depending on the site to be applied or the function, for scalp and hair care cosmetic preparations, partial cosmetic preparations (e.g., eye makeup cosmetic preparations, lip cosmetic preparations, oral cosmetic preparations, and nail cosmetic preparations), in addition, tanning or sunburn cosmetic preparations, cosmetic preparations for pigmented spot and fleck, cosmetic preparations for acne, deodorant cosmetic preparations and others. Further, the composition is useful for various foods, in addition, functional foods, for example, health foods, health supplements, foods for specified uses, and foods with health claims. Furthermore, the composition is useful for animal feeding stuffs for domestic animals (e.g., cattle, pigs, and sheep), pet animals (e.g., mammals such as dogs and cats, birds (or poultry), and reptiles), fish (e.g., cultured fish such as sea breams and eels; and aquarium fish such as goldfish), and experimental (or laboratory) animals (e.g., rats)].

### EXAMPLES

Hereinafter, the following examples are intended to describe this invention in further detail and should by no means be interpreted as defining the scope of the invention.

### Example 1

A peptide chain represented by the formula: H-(Pro-Hyp-Gly)₄-Glu-Hyp-Gly-(Pro-Hyp-Gly)₅-OH (Sequence ID: 1) was synthesized by a solid-phase synthesis with an automatic peptide synthesis machine. That is, with the use of 0.1 mmol of a particulate resin [HMP glycine, manufactured by Applied Biosystems (US)] which comprised a styrene-divinylbenzene copolymer [molar ratio of styrene relative to divinylbenzene: 99/1] containing 4-(N^{α}-9-(fluorenylmethoxycarbonyl)-glycine)-oxymethyl-p henoxy-methyl group in a substitution of 0.65 mmol/g (resin), the carboxyl terminal of one amino acid was sequentially linked (or bound) to the amino terminal of the other amino acid so as to give an object peptide. In this link reaction, 1 mmol of N^{α}-9- (fluorenylmethoxycarbonyl) -L-proline [Fmoc proline], 1 mmol of N^{α}-9-(fluorenylmethoxycarbonyl)-glycine [Fmoc glycine], and 1 mmol of N^{α}-9-(fluorenylmethoxycarbonyl)-γ-t-butyloxy-L-glutamic acid [Fmoc glutamic acid] (each manufactured by Applied Biosystems (US)) and 1 mmol of N^{α}-9-(fluorenylmethoxycarbonyl)-O-t-butyl-L-hydroxyproline [Fmoc hydroxyproline] (manufactured by Bachem AG) were used as amino acids in each linking step.

The obtained peptide resin was treated with 10 mL of trifluoroacetic acid containing 5% water for 3 hours. The resulting solution was added to diethyl ether to form a precipitate, and the precipitate was further washed with diethyl ether several times to deprotect the peptide and to eliminate the peptide from the resin. The resulting crude product was purified by a PD10 column (manufactured by Amarsham Bioscience K.K.) to give a peptide. The purified peptide obtained in the foregoing manner was subjected to a column chromatography ["AKTA explorer10XT" manufactured by Amarsham Bioscience K.K., column: "Nova-Pak C18", manufactured by Millipore Corporation, 3.9 mmφ x 150 mm, mobile phase: a mixed solvent of acetonitrile and water containing 0.05 vol.% of trifluoroacetic acid (concentration of acetonitrile was linearly increased from 5 to 50 vol.% for 30 minutes), flow rate: 1.0 mL/min.], and a single peak was shown at a retention time of 11.4 minutes. The molecular weight of the purified peptide was determined as 2722 based on FAB method mass spectrum (theoretical value: 2722.9).

A peptide (2.5 mg (0.0009 mmol)) represented by the formula:
H-(Pro-Hyp-Gly)₄-Glu-Hyp-Gly-(Pro-Hyp-Gly)₅-OH
(Sequence ID: 1) was dissolved in 0.25 mL of 10 mM phosphate buffer (pH 7.4) with stirring at a room temperature. To the mixture were added 0.12 mg (0.0009 mmol) of 1-hydroxybenzotriazole and 1.7 mg (0.0092 mmol) of 1-ethyl-3-(3-dimethylaminopropyl)-carbodiimide hydrochloride, and the resulting mixture was further stirred for 2 days at 20°C. The obtained reaction solution was diluted 10-fold with water, and the diluted solution was dialyzed against water for 3 days for removing a reagent (such a condensing agent) and an unreacted monomer to give a polypeptide of the present invention. The proportion of the peptide unit (1) relative to the peptide unit (2) [(1)/(2)] was 90/10 (molar ratio).

The obtained polypeptide was subjected to a gel-permeation chromatography (AKTA purifier system, manufactured by Amarsham Bioscience K.K., column: Superose 6 HR GL, flow rate: 0.5 mL/min., eluent: 10 mM phosphate buffer (pH 7.4) containing 150 mM NaCl), and the peak of the molecular weight of the polypeptide was confirmed in the range from 100, 000 to 2, 000, 000 in the molecular weight distribution. The molecular weight was calculated with a Gel Filtration HMW Calibration Kit (manufacturedbyAmarsham Bioscience K.K.) as a reference material.

The circular dichroism spectrum of the obtained polypeptide was measured, and positive Cotton effect was observed at a wavelength of 225 nm and negative Cotton effect at a wavelength of 197 nm. The results confirmed that the polypeptide formed a triple helical structure.

### Example 2

A peptide (1.2 mg (0.00045 mmol)) represented by the formula: H-(Pro-Hyp-Gly)₁₀-OH (Sequence ID: 2; manufactured by Peptide Institute, Inc.) and a peptide (1.2 mg (0.00045 mmol)) represented by the formula:
H-(Pro-Hyp-Gly)₄-Glu-Hyp-Gly-(Pro-Hyp-Gly)₅-OH
(Sequence ID: 1) that was obtained in Example 1 were dissolved in 0.25 mL of 10 mM phosphate buffer solution (pH 7.4). To the peptide solution were added 0.12 mg (0.0009 mmol) of 1-hydroxybenzotriazole and 15.7 mg (0.082 mmol) of 1-ethyl-3-(3-dimethylaminopropyl)-carbodiimide hydrochloride, and the resulting mixture was further stirred for 2 days at 20°C. The reaction solution was diluted 10-fold with water, and the diluted solution was dialyzed against water for 3 days for removing a reagent (such as a condensing agent) and an unreacted monomer to give a polypeptide of the present invention. The proportion of the peptide unit (1) relative to the peptide unit (2) [(1)/(2)] was 95/5 (molar ratio).

The resulting polypeptide was subjected to a gel-permeation chromatography (AKTA purifier system, manufactured by Amarsham Bioscience K.K., column: Superose 6 HR GL, flow rate: 0.5 mL/min., eluent: 10 mM phosphate buffer (pH 7.4) containing 150 mM NaCl), and the peak of the molecular weight of the polypeptide was confirmed in the range from 140, 000 to 2, 000, 000 in the molecular weight distribution. The molecular weight was calculated with a Gel Filtration HMW Calibration Kit (manufacturedbyAmarsham Bioscience K.K.) as a reference material.

The circular dichroism spectrum of the obtained polypeptide was measured, and positive Cotton effect was observed at a wavelength of 224 nm and negative Cotton effect at a wavelength of 196 nm. The results confirmed that the polypeptide formed a triple helical structure.

### Comparative Example 1

A peptide (2.4 mg (0.0009 mmol)) represented by the formula: H-(Pro-Hyp-Gly)₁₀-OH (Sequence ID: 2; manufactured by Peptide Institute, Inc.) was suspended in 1 mL of dimethyl sulfoxide with stirring at a room temperature. To the mixture were added 0.12 mg (0.0009 mmol) of diisopropylethylamine, 0.12 mg (0.0009 mmol) of 1-hydroxybenzotriazole, and 0.34 mg (0.0018 mmol) of 1-ethyl-3-(3-dimethylaminopropyl)-carbodiimide hydrochloride, and the resulting mixture was further stirred for 2 days at 20°C. The obtained reaction solution was diluted 3-fold with water, and the diluted solution was dialyzed against water for 3 days for removing a reagent (such as a condensing agent) and an unreacted monomer to give a polypeptide. The proportion of the peptide unit (1) relative to the peptide unit (2) [(1)/(2)] was 100/0 (molar ratio).

The resulting polypeptide was subjected to a gel-permeation chromatography (AKTA purifier system, manufactured by Amarsham Bioscience K.K., column: Superose 6 HR GL, flow rate: 0.5 mL/min., eluent: 10 mM phosphate buffer (pH 7.4) containing 150 mM NaCl), and the peak of the molecular weight of the polypeptide was confirmed in the range from 140,000 to 600,000 in the molecular weight distribution. The molecular weight was calculated with a Gel Filtration HMW Calibration Kit (manufacturedbyAmarsham Bioscience K.K.) as a reference material.

The circular dichroism spectrum of the obtained polypeptide was measured, and positive Cotton effect was observed at a wavelength of 224 nm and negative Cotton effect at a wavelength of 196 nm. The results confirmed that the polypeptide formed a triple helical structure.

### Example 3

Using polypeptides obtained in Examples 1 to 2 and a polypeptide obtained in Comparative Example 1, each polypeptide was dissolved in water to obtain an aqueous solution having a concentration of about 0.5 mg/ml. The three obtained aqueous solutions were used for the following experiment, respectively. One volume of the aqueous solution was mixed to two volumes of an aqueous solution that contained Na⁺ (213 mM), K⁺ (7.5 mM), Ca²⁺ (3.8 mM), Mg ²⁺ (2.3 mM), Cl⁻ (223.3 mM), HCO₃⁻ (6.3 mM), HPO₄²⁻ (1.5 mM), and SO₄²⁻ (0.75 mM) and adjusted to pH 7.4 with a Tris buffer (final concentration, Na⁺: 142 mM, K⁺: 5 mM, Ca²⁺: 2.5 mM, Mg ²⁺ : 1.5 mM, Cl⁻: 148.8 mM, HCO₃⁻: 4.2 mM, HPO₄²⁻: 1 mM, and SO₄²⁻: 0.5 mM), and thus the polypeptide was allowed to stand in the solution at 36.5°C for 1 day. During this time, a small amount of the solution was collected as needed and allowed to pass through a PsD-10 column (manufactured by Amarsham Bioscience K.K.) for removing salts therefrom. Then, each solution from which the salts had been removed was subjected to a negative staining with phosphotungstic acid and observed by a transmission electron microscope (H-7600 type, manufactured by Hitachi, Ltd.).

In the polypeptide obtained in Example 1, after 24 hours a fiber assembly structure having a diameter of about 10 nm and an apatite crystal formed on a surface of the structure were observed as shown in Fig. 1. Moreover, in the polypeptide of Example 2, the similar image was observed. On the other hand, in the polypeptide of Comparative Example 1, a fiber assembly having a diameter of about 10 nm was observed, but no apatite was observed.

**Formulation Example 1 (nutrition supplement drink)**

| | |
|---|---|
| Freeze-dried polypeptide of Example 1 | 0.02 g |
| Vitamin C 0.1 | g |
| Calcium lactate | 0.01 g |
| Glucose | 10 g |
| Citric acid | 1 g |
| Purified water | 100 mL |

**Formulation Example 2 (nutriceutical food)**

| | |
|---|---|
| Freeze-dried polypeptide of Example 1 | 0.05 g |
| Vitamin C 0.1 | g |
| Calcium lactate | 0.1 g |
| Sucrose fatty acid ester | 0.1 g |

**Formulation Example 3 (skin lotion)**

| | |
|---|---|
| Freeze-dried polypeptide of Example 1 | 0.1 g |
| Polyethylene glycol (PEG1500) | 0.1 g |
| Propylene glycol | 0.5 g |
| Glycerin | 0.5 g |
| Purified water | 10 mL |

To the mixture of the above-mentioned components is added 1 mL of an ethanol solution having 1% by weight of paraben and stirred thoroughly. Then, the resulting mixture is made to 20 mL with purified water.

**Formulation Example 4 (milky lotion)**

| | |
|---|---|
| Freeze-dried polypeptide of Example 1 | 0.5 g |
| Propylene glycol | 5.0 g |
| Polyethylene glycol | 3.0 g |
| Triethanolamine | 1.0 g |
| Purified water | 70 g |

The mixture of the above-mentioned components is heated, and the temperature of the mixture is adjusted to 70°C (Mixture A).

On the other hand, a mixture of 2.0 g of stearic acid, 1.0 g of cetyl alcohol, 2.0 g of petrolatum, 5.0 g of squalan, and 2.0 g of glycerol tri-2-ethylhexanoic acid ester is heated to 75°C, and 2.0 g of sorbitan monooleic acid ester and 0.1 g of the paraben are added thereto. The temperature of the resulting mixture is adjusted to 70°C. The obtained mixture (Mixture B) is added to the Mixture A, stirred, mixed, and further processed in a homogenizer to homogenize emulsified particles. The resulting mixture is deaerated and filtered, and the filtrate is cooled to give a milky lotion.

### Formulation Example 5 (moisturizing essence)

To 14 g of purified water are added 0.4 g of the freeze-dried polypeptide of Example 1, 1.6 g of sorbitol, 1. 0 g of propylene glycol, and 1.4g of a polyethylene glycol (PEG1500) to prepare an aqueous solution. Separately, to 1.4 g of ethanol are added 0.2 g of a polyoxyethylene oleyl alcohol ether, 40 mg of an olive oil, and 20 mg of paraben to prepare an ethanol solution. The aqueous solution is added and mixed to the ethanol solution to prepare an essence.

### Formulation Example 6 (hand cream)

In 66.2g of purified water, 0.5g of the freeze-dried polypeptide of Example 1, 15.0 g of glycerin, 3.0 g of propylene glycol, and 0.2 g of potassium hydroxide are dissolved. The resulting solution is heated, and the temperature of the solution is adjusted to 70°C to prepare Solution A.

On the other hand, 3.0 g of stearic acid, 3.0 g of monoglyceride stearate, 3.0 g of petrolatum, 6.0 g of liquid paraffin, and 0.1 g of paraben are mixed and heated, and the temperature of the mixture is adjusted to 70°C. The resulting mixture is added to the Solution A, mixed with stirring, and processed in a homogenizer to homogenize emulsified particles. The obtained mixture is deaerated and filtered. The filtrate is cooled to give a hand cream.

## Claims

1. A novel polypeptide containing a peptide unit having an amino acid sequence represented by the following formula (1) and a peptide unit having an amino acid sequence represented by the following formula (2):
- Pro-X-Gly- (1)
- Y-Z-Gly- (2)
wherein "X" and "Z" are the same or different, each representing Pro or Hyp, and "Y" represents an amino acid residue having a carboxyl group.

2. A polypeptide according to claim 1, wherein, in the formula (2), "Y" is Asp, Glu, or Glu having a further carboxyl group at γ-position.

3. A polypeptide according to claim 1, wherein the proportion (molar ratio) of the peptide unit (1) relative to the peptide unit (2) [(1)/(2)] is 99/1 to 1/99.

4. A polypeptide according to claim 1, which shows positive Cotton effect at a wavelength in the range of 220 to 230 nm and negative Cotton effect at a wavelength in the range of 195 to 205 nm in a circular dichroism spectrum.

5. A polypeptide according to claim 1, wherein at least part of the polypeptide is capable of forming a triple helical structure.

6. A polypeptide according to claim 1, which shows a peak corresponding to the molecular weight in the range from 5 x 10³ to 500 x 10⁴.

7. A polypeptide according to claim 1, which is capable of forming a collagenous tissue.

8. A polypeptide according to claim 1, which has an apatite supported thereon.

9. A process for producing a polypeptide recited in claim 1, which comprises condensing an amino acid component or peptide fragment component, wherein the component at least contains an amino acid or peptide fragment corresponding to the formula (1) and an amino acid or peptide fragment corresponding to the formula (2) recited in claim 1.

10. A process for producing a polypeptide recited in claim 1, which comprises condensing
(a) a peptide component at least containing a peptide having both amino acid sequences represented by the formulae (1) and (2) recited in claim 1, or
(b) a peptide component at least containing a peptide having an amino acid sequence represented by the formula (1) and a peptide having an amino acid sequence represented by the formula (2).

11. A process for producing a polypeptide having an apatite supported thereon, which comprises
bringing a polypeptide recited in claim 1 into contact with an aqueous solution containing a calcium ion and a phosphate ion to deposit or precipitate an apatite on the polypeptide.

12. A process according to claim 11, wherein the apatite comprises a hydroxyapatite.

13. A polypeptide having an apatite supported thereon, which is obtainable by a production process recited in claim 11 or 12.

14. A composition containing a polypeptide recited in claim 1 or 13.

15. A composition according to claim 14, which is a cosmetic preparation or a food composition.
